# EUROPÄISCHE PATENTSCHRIFT

(11) **EP 3 248 635 B1**
(45) Veröffentlichungstag und Bekanntmachung des Hinweises auf die Patenterteilung: **29.01.2020**
(21) Anmeldenummer: 17166983.1
(22) Anmeldetag: 19.04.2017
(51) Int. Cl.: A61M 5/178, A61M 5/315, A61M 5/31, A61M 25/00

(54) **KOMPONENTE FÜR EINE SPRITZE SOWIE SPRITZE**
COMPONENT FOR A SYRINGE AND SYRINGE
COMPOSANT POUR UN SERINGUE ET SERINGUE

(30) Priorität: 24.05.2016 DE 102016109505
(43) Veröffentlichungstag der Anmeldung: 29.11.2017
(73) Patentinhaber: Gerresheimer Regensburg GmbH, 93047 Regensburg (DE)
(72) Erfinder: PFRANG, Jürgen, 93183 Kallmünz (DE); SCHMITT, Bernhard, 92442 Wackersdorf (DE)
(74) Vertreter: Reichert & Lindner Partnerschaft Patentanwälte

(56) Entgegenhaltungen:
- EP-A1- 1 064 038
- EP-A1- 3 192 547
- WO-A1-2015/151671
- JP-A- 2003 180 832
- JP-A- 2006 288 652
- US-A- 2 618 264
- US-A1- 2014 228 746

## Beschreibung

Die Erfindung betrifft eine Komponente für eine Spritze und eine Spritze. Insbesondere betrifft die Erfindung eine Komponente für eine Spritze, welche reibungsvermindernd ausgestaltet ist.

Spritzen finden vorwiegend im medizinischen Bereich Anwendung und umfassen bekanntlich einen Zylinder, in welchem ein Kolben beweglich angebracht ist. Ferner kann eine Dichtung zwischen Zylinder und Kolben vorgesehen sein; die Dichtung kann auch ein Ende des Kolbens bilden, etwa in Form eines Stopfens aus Gummi oder anderem elastischen Material. Durch Bewegung des Kolbens im Zylinder kann eine Wirkstoffzubereitung z.B. einem Patienten verabreicht, oder eine Probe, etwa eine Blutprobe, einem Patienten entnommen werden, jeweils über eine an der Spritze angebrachte Kanüle. Hierbei ist es erstrebenswert, dass die Bewegung des Kolbens im Zylinder möglichst reibungsarm erfolgt. Zu große Reibung kann bei Kraftausübung auf den Kolben zu ruckartigen Bewegungen des Kolbens führen. Solch eine ruckartige Bewegung verhindert eine genaue Dosierung bei Verabreichung einer Wirkstoffzubereitung ebenso wie bei der Probenentnahme und führt zu zusätzlicher Belastung des Patienten, da eine ruckartige Bewegung des Kolbens oftmals auch zusätzliche Bewegungen der Kanüle im Körper des Patienten zur Folge hat.

Zur Verminderung der Reibung zwischen Kolben und Zylinder oder zwischen anderen Spritzenkomponenten ist es bekannt, eine zusätzliche Schicht mit reibungsmindernden Eigenschaften, etwa eine Silikonschicht oder eine PTFE-Schicht, zwischen Kolben und Zylinder vorzusehen, wie beispielsweise aus JP 2006 288652 A und auch aus WO 2015/151671 A1 bekannt. Auch deutlich voneinander beabstandete, angebrachte Flansche und Rippen, wie beispielsweise aus EP 1 064 038 A1 und JP 2003 180832 A bekannt, separat aufgebrachte schwammähnliche Strukturelemente, wie aus US 2014/228746 A1 bekannt, oder Aussparungen für Schmiermittel, wie aus US 2 618 264 A bekannt, reduzieren die Reibung und gewährleisten gleichzeitig eine gewisse Dichtigkeit zwischen zwei Reibpartnern.

Die europäische Patentanmeldung EP 2 653 484 A1 beschreibt, unter anderem im Hinblick auf Spritzen, die chemische Veränderung einer Oberfläche zum Zwecke der Reibungsreduzierung. Hierzu wird auf der Oberfläche aus einem Monomer eine Polymerschicht erzeugt. Ein ähnliches Verfahren wird in der europäischen Patentschrift EP 2 752 436 B1 beschrieben.

EP 3 192 547 A1 offenbart Scheiben als Strukturelemente, die quer zu einer Längsachse einer Kolbenstange einer Spritze angeordnet sind und dem Vermeiden des Verkantens der Kolbenstange dienen. Rippen stellen weitere Strukturelemente dar, die in axialer Richtung entlang der Längsachse der Kolbenstange angeordnet sind, also quer und in anderer Richtung als die Scheiben. Auch die Längsrippen-Strukturelemente dienen ausschließlich der Versteifung der Kolbenstange.

Eine Schicht aus einem Material wie Silikon kann aber bei manchen Wirkstoffen, z.B. Biosimilars, zu pharmazeutisch unerwünschten Wechselwirkungen zwischen dem Material der Schicht und dem Wirkstoff führen. Manche Materialien wie PTFE zeigen zwar eine wünschenswerte reibungsmindernde Wirkung, sind aber zugleich relativ hart, so dass darunter die Dichtigkeit leidet, d.h. der Kolben schließt in diesem Fall nicht ausreichend dicht mit dem Zylinder ab.

Es ist daher Aufgabe der Erfindung, eine Komponente für eine Spritze bereitzustellen, so dass einerseits der Zylinder ausreichend dicht mit dem Kolben respektive der Dichtung abschließt, und zugleich die Reibung zwischen dem Zylinder und dem Kolben, respektive zwischen dem Zylinder und der Dichtung gegenüber dem Stand der Technik reduziert ist, ohne dass es zu pharmazeutisch unerwünschten Wechselwirkungen kommt.

Diese Aufgabe wird gelöst durch eine Komponente für eine Spritze gemäß Anspruch 1. Vorteilhafte Ausgestaltungen der Erfindung finden sich in den Unteransprüchen.

Die erfindungsgemäße Komponente für eine Spritze umfasst die Merkmale des Anspruchs 1 und weist eine Oberflächenstruktur auf, welche durch Formgebung eines Bereiches der Komponente gebildet ist. Die Oberflächenstruktur ist dazu ausgebildet, durch ihre Form in der Spritze reibungsreduzierend zu wirken. Dies bedeutet, die Form ist derart, dass sich, wenn die Komponente in die Spritze in der vorgesehenen, an sich bekannten Weise eingefügt wird, die Komponente in der Spritze mit einer Reibung gegenüber mindestens einer anderen Komponente bewegen lässt, welche im Vergleich zur Verwendung einer Komponente ohne die genannte Oberflächenstruktur reduziert ist.

Der große Vorteil einer erfindungsgemäßen Komponente ist, dass eine gewünschte Reduzierung der Reibung erzielt werden kann, ohne ein zusätzliches Material in die Spritze einzubringen. Auf diese Weise werden pharmazeutisch unerwünschte Wirkungen eines zusätzlichen Materials auf einen zu verabreichenden Wirkstoff oder eine zu entnehmende Probe zuverlässig ausgeschlossen.

Die Komponente für eine Spritze ist in Ausführungsformen der Erfindung ein Zylinder für die Spritze, oder ein Kolben für die Spritze, oder eine Dichtung für einen Kolben der Spritze.

Ist die Komponente ein Zylinder für eine Spritze, so befindet sich die Oberflächenstruktur an einer Innenseite eines Mantels des Zylinders. Auf diese Weise kann die Reibung an einem Kolben bzw. einer Dichtung für den Kolben der Spritze durch die Oberflächenstruktur verringert werden.

Ist die Komponente ein Kolben für eine Spritze, so ist die Oberflächenstruktur so an dem Kolben angeordnet, dass sie in der Spritze, das heißt bei bestimmungsgemäßer Einführung des Kolbens in die Spritze, einem Mantel eines Zylinders für die Spritze gegenüberliegt. Auf diese Weise kann die Reibung an einem Zylinder für die Spritze durch die Oberflächenstruktur verringert werden.

Ist die Komponente eine Dichtung für einen Kolben einer Spritze, so ist die Oberflächenstruktur so an der Dichtung angeordnet, dass sie in der Spritze, das heißt bei bestimmungsgemäßer Einführung des die Dichtung tragenden Kolbens in die Spritze, einem Mantel eines Zylinders für die Spritze gegenüberliegt. Auf diese Weise kann die Reibung an einem Zylinder für die Spritze durch die Oberflächenstruktur verringert werden.

Die Oberflächenstruktur weist eine Vielzahl von Strukturelementen auf. Dabei können die Strukturelemente gleichartig oder unterschiedlich sein.

Insbesondere ist in einer Ausführungsform jedes solche auf der Komponente vorhandene Strukturelement von einer der folgenden Arten, und zwar unabhängig von den übrigen auf der Komponente vorhandenen Strukturelementen: linienförmige Eintiefung, linienförmige Erhöhung, punktförmige Eintiefung, punktförmige Erhöhung, Eintiefung mit elliptischem Grundriss, Erhöhung mit elliptischem Grundriss, Eintiefung mit polygonalem Grundriss, Erhöhung mit polygonalem Grundriss. In diesem Zusammenhang beinhaltet elliptisch insbesondere den Spezialfall kreisförmig.

In bestimmten Ausführungsformen sind die Strukturelemente regelmäßig an der erfindungsgemäßen Komponente angeordnet. In anderen Ausführungsformen sind die Strukturelemente unregelmäßig an der erfindungsgemäßen Komponente angeordnet.

Insbesondere können die Strukturelemente so an der erfindungsgemäßen Komponente angeordnet sein, dass ein Abstand zwischen benachbarten Strukturelementen im Bereich 10 µm bis 1 mm liegt. Es ist hierbei in Ausführungsformen möglich, dass die Strukturelemente regelmäßig angeordnet sind, und der Abstand zwischen benachbarten Strukturelementen einen festen Wert aus dem genannten Bereich hat. Ebenso ist es etwa möglich, dass die Anordnung der Strukturelemente unregelmäßig ist, und der Abstand zwischen benachbarten Strukturelementen nicht konstant ist, sondern, je nach betrachtetem Paar benachbarter Strukturelemente, verschiedene Werte aus dem genannten Bereich annimmt.

Eine Breite eines Strukturelements liegt in Ausführungsformen im Bereich 10 µm bis 100 µm. Ähnlich wie im Falle des Abstandes ist es hierbei möglich, dass alle Strukturelemente einen gleichen Wert aus dem genannten Bereich für die Breite aufweisen, oder aber, dass die Strukturelemente verschiedene Werte für die Breite aufweisen, welche in dem genannten Bereich liegen.

Ist das Strukturelement eine Erhöhung, so liegt eine Höhe des Strukturelements in Ausführungsformen im Bereich 1 µm bis 100 µm. Ist das Strukturelement eine Eintiefung, so liegt eine Tiefe des Strukturelements in Ausführungsformen im Bereich 1 µm bis 100 µm. Ähnlich wie im Falle des Abstandes ist es hierbei möglich, dass alle Strukturelemente einen gleichen Wert aus dem genannten Bereich für die Höhe bzw. Tiefe aufweisen, oder aber, dass die Strukturelemente verschiedene Werte für die Höhe bzw. Tiefe aufweisen, welche in dem genannten Bereich liegen.

In Ausführungsformen sind die Strukturelemente in bestimmten Mustern auf der Komponente angeordnet. Insbesondere können dabei die Strukturelemente so auf der Komponente angeordnet sein, dass sie bezüglich einer Bewegungsrichtung des Kolbens im Zylinder der Spritze parallel oder senkrecht oder in einem Winkel zwischen 30 Grad und 60 Grad liegen.

In Ausführungsformen umfasst die Oberflächenstruktur auf der Komponente mindestens zwei Gruppen von Strukturelementen. Die Strukturelemente innerhalb einer Gruppe sind dabei gleichartig. Die Strukturelemente zweier der Gruppen unterscheiden sich hinsichtlich wenigstens eines der folgenden, vorstehend bereits diskutierten Merkmale: Form, Breite, Höhe, Tiefe, Ausrichtung bezüglich einer Bewegungsrichtung des Kolbens im Zylinder der Spritze.

Eine erfindungsgemäße Spritze umfasst mindestens eine erfindungsgemäße Komponente der vorstehend beschriebenen Art. Auf diese Weise ist die Reibung zwischen Komponenten der Spritze, also zwischen Zylinder und Kolben oder zwischen Zylinder und Dichtung für den Kolben, verringert.

In einer besonderen Ausführung sind nicht nur zwei Komponenten der Spritze erfindungsgemäße Komponenten der vorstehend beschriebenen Art, sondern die jeweiligen auf der Komponente erfindungsgemäß vorhandenen Oberflächenstrukturen sind derart, dass sich eine zusätzliche reibungsreduzierende Wirkung aus dem Zusammenwirken der jeweiligen Oberflächenstrukturen der Komponenten ergibt.

Spritzen bestehen im Allgemeinen aus Kunststoff. Die beschriebenen Oberflächenstrukturen können bei der Herstellung der jeweiligen Komponente der Spritze beispielsweise durch geeignete Formen in einem Spritzgussverfahren oder durch Pressen mit einem entsprechend geformten Formwerkzeug hergestellt werden, ohne dass die Erfindung auf durch die genannten Herstellungsweisen gefertigte Komponenten für Spritzen beschränkt ist.

Im Folgenden werden die Erfindung und ihre Vorteile noch an Hand spezifischer Ausführungsbeispiele und zugehöriger schematischer Zeichnungen näher erläutert. Die in den Zeichnungen dargestellten und in der zugehörigen Figurenbeschreibung erläuterten Ausführungsformen stellen dabei lediglich Beispiele dar, wie die erfindungsgemäße Komponente für eine Spritze ausgestaltet sein kann, und sind nicht als Beschränkung der Erfindung aufzufassen.
- **Fig. 1A und 1B**: zeigen schematische Ausführungen von Spritzen, bei denen die vorliegende Erfindung vorteilhaft eingesetzt werden kann.
- **Fig. 2 bis 4**: zeigen Beispiele für Komponenten für eine Spritze gemäß der Erfindung versehen mit einer Oberflächenstruktur.
- **Fig. 5 bis 8**: zeigen Beispiele einer Oberflächenstruktur, wie sie auf einer erfindungsgemäßen Komponente ausgeformt sein kann, in Draufsicht.
- **Fig. 9 bis 12**: zeigen weitere Beispiele einer Oberflächenstruktur, wie sie auf einer erfindungsgemäßen Komponente ausgeformt sein kann, in Draufsicht.
- **Fig. 13 bis 15**: zeigen weitere Beispiele einer Oberflächenstruktur, wie sie auf einer erfindungsgemäßen Komponente ausgeformt sein kann, in Draufsicht.
- **Fig. 16 bis 19**: zeigen Schnittansichten durch verschiedene Strukturelemente einer Oberflächenstruktur, wie sie auf einer erfindungsgemäßen Komponente ausgeformt sein kann.
- **Fig. 20 und 21**: zeigen eine Draufsicht auf Beispiele einer Kombination von Oberflächenstrukturen, wie sie auf einer erfindungsgemäßen Komponente ausgeformt sein können.
- **Fig. 22**: zeigt eine Draufsicht auf ein weiteres Beispiel einer Kombination von Oberflächenstrukturen, wie sie auf einer erfindungsgemäßen Komponente ausgeformt sein können.

In den Zeichnungen sind zwecks Verdeutlichung manche Elemente nicht maßstabsgetreu dargestellt. Gleiche Bezugszeichen werden für gleiche oder entsprechende Elemente der Erfindung in verschiedenen Ausführungsformen verwendet.

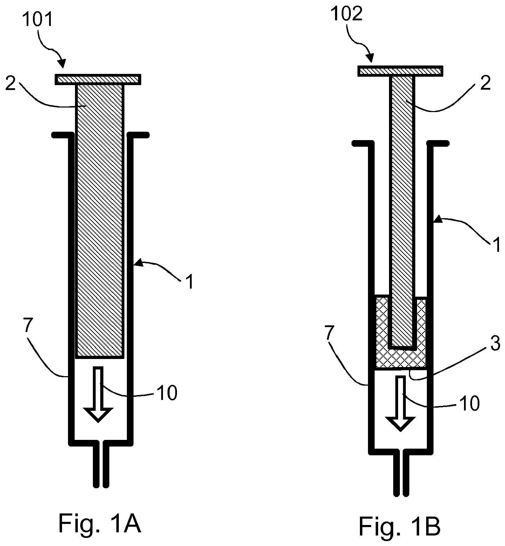

**Fig. 1A und 1B** zeigen schematisch den an sich bekannten Aufbau zweier Spritzen 101 und 102. In **FIG. 1A****,** umfasst die Spritze 101 einen Zylinder 1, in welchem ein Kolben 2 beweglich angeordnet ist. Der Zylinder 1 hat insbesondere einen Mantel 7. In **FIG. 1B****,** umfasst die Spritze 102 einen Zylinder 1, in welchem ein Kolben 2 beweglich angeordnet ist. Eine Dichtung 3 ist am Kolben 2 angebracht, um den Kolben 2 gegen den Zylinder 1 abzudichten. Zylinder 1, Kolben 2, und Dichtung 3 bilden die Komponenten der Spritze 101 bzw. 102 im Sinne dieser Anmeldung. Zur Verabreichung eines Wirkstoffes oder zur Entnahme einer Probe wird der Kolben 2 in an sich bekannter Weise jeweils relativ zum Zylinder 1 bewegt, entlang der durch den Pfeil angezeigten Richtung 10 bzw. entgegengesetzt dazu. Dabei wirken im Falle der Spritze 101 zwischen Kolben 2 und Zylinder 1, und im Falle der Spritze 102 zwischen Dichtung 3 und Zylinder 1 Reibungskräfte. Diese Reibungskräfte können beispielsweise die Verabreichung eines Wirkstoffs oder die Entnahme einer Probe erschweren, wie oben bereits erwähnt. Bei Komponenten 1, 2, 3 der Spritze 101 bzw. 102 gemäß der Erfindung sind diese Reibungskräfte reduziert, ohne ein zusätzliches, pharmazeutisch mitunter unerwünschtes, Material in die Spritze 101, 102 einbringen zu müssen.

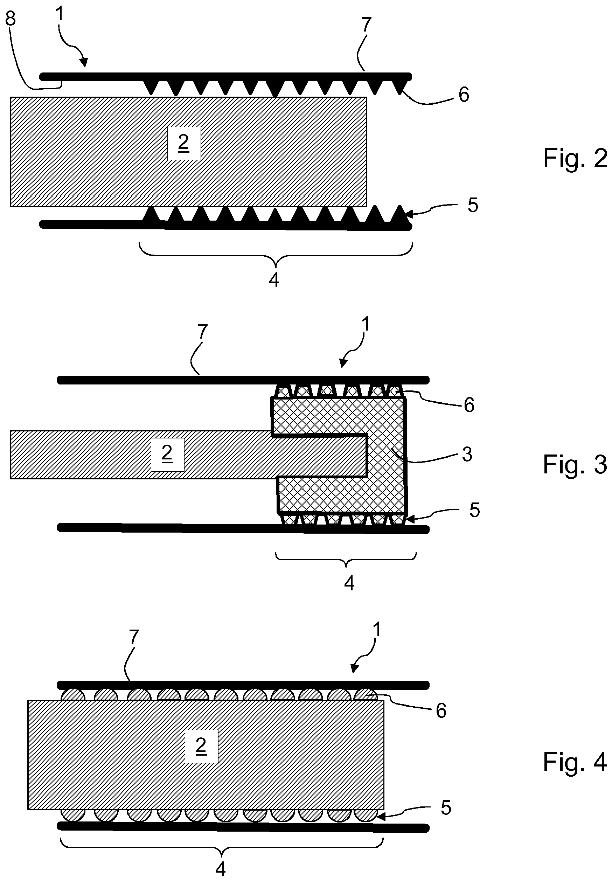

**Fig. 2 bis 4** zeigen drei Beispiele, wie erfindungsgemäße Komponenten einer Spritze ausgestaltet sein können. Eine erfindungsgemäße Komponente weist einen Bereich 4 mit einer Oberflächenstruktur 5 auf. In den gezeigten Beispielen besteht jede Oberflächenstruktur 5 aus einer Vielzahl von Strukturelementen 6. Kolben 2 und Zylinder 1 sind dabei jeweils nur teilweise gezeigt.

Im Beispiel der **Fig. 2** ist ein Bereich 4 des Zylinders 1 als Oberflächenstruktur 5 ausgeformt. Die Reibung zwischen dem Kolben 2 und dem Zylinder 1 ist dadurch verringert. Die Oberflächenstruktur 5 ist an einer Innenseite 8 eines Mantels 7 des Zylinders 1 ausgeformt. Es ist hier ein Kolben 2 ohne Dichtung gezeigt. Ebenso könnte ein Zylinder 1, wie in **Fig. 2** gezeigt, auch bei einer Spritze 102 (siehe **Fig. 1B**) mit einer Dichtung 3 für den Kolben 2 eingesetzt werden.

Im Beispiel der **Fig. 3** ist ein Bereich 4 einer Dichtung 3 für den Kolben 2 als Oberflächenstruktur 5 ausgeformt. Die Reibung zwischen der Dichtung 3 und dem Zylinder 1 ist dadurch verringert. Der Kolben 2 mit der Dichtung 3 ist hier in einen Zylinder 1 einer Spritze 102 (siehe **Fig. 1B**) eingesetzt gezeigt. Der Bereich 4 der Dichtung 3, der als Oberflächenstruktur 5 ausgeformt ist, liegt einem Mantel 7 des Zylinders 1 gegenüber.

Im Beispiel der **Fig. 4** ist ein Bereich 4 des Kolbens 2 als Oberflächenstruktur 5 ausgeformt. Die Reibung zwischen dem Kolben 2 und dem Zylinder 1 ist dadurch verringert. Der Kolben 2 ist hier in einen Zylinder 1 einer Spritze 101 (siehe **Fig. 1A**) eingesetzt gezeigt. Der Bereich 4 des Kolbens 2, der als Oberflächenstruktur 5 ausgeformt ist, liegt einem Mantel 7 des Zylinders 1 gegenüber.

In allen Fällen ist es ein Bereich 4 der jeweiligen erfindungsgemäßen Komponente, also Zylinder 1 im Falle der **Fig. 2****,** Dichtung 3 im Falle der **Fig. 3****,** und Kolben 2 im Falle der **Fig. 4****,** welcher als die Oberflächenstruktur 5 ausgebildet ist. Ein weiteres Material wird somit nicht in die Spritze eingebracht.

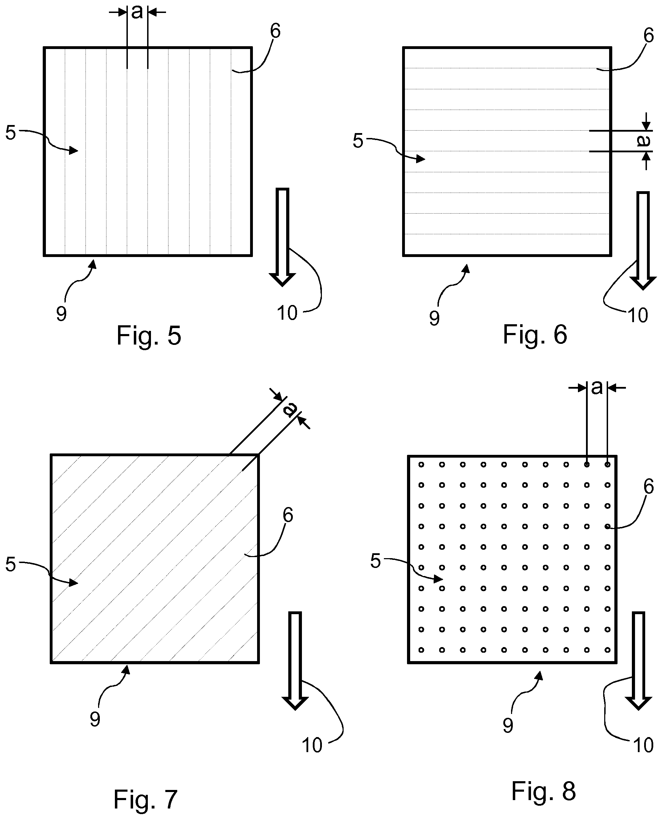

**Fig. 5 bis 8** zeigen in Draufsicht Beispiele für eine Oberflächenstruktur 5, wie sie auf einer Komponente 1, 2, 3 ausgeformt sein kann. Jede Oberflächenstruktur 5 besteht aus einer Vielzahl von Strukturelementen 6. Es ist hier jeweils nur ein Ausschnitt 9 einer Komponente gezeigt, wobei es unerheblich ist, ob es sich bei der Komponente um einen Zylinder 1, einen Kolben 2 oder eine Dichtung 3 handelt. Der Pfeil zeigt jeweils die bereits in **Fig. 1A und 1B** gezeigte Richtung 10 an.

In den Beispielen der **Fig. 5, Fig. 6** und **Fig. 7** sind die Strukturelemente 6 linienförmig; es können linienförmige Erhöhungen oder linienförmige Eintiefungen sein. Im Beispiel der **Fig. 8** sind die Strukturelemente 6 punktförmig; es können punktförmige Erhöhungen oder punktförmige Eintiefungen sein. In den in den **Fig. 5 bis 8** gezeigten Beispielen ist die Anordnung der Strukturelemente 6 regelmäßig. Die Anordnung der Strukturelemente 6 ist insbesondere durch einen Abstand a zwischen benachbarten Strukturelementen 6 gekennzeichnet. Wie aus den Figuren ersichtlich, sind im Falle der **Fig. 5** die linienförmigen Strukturelemente 6 parallel zu der Richtung 10 ausgerichtet, im Falle der **Fig. 6** senkrecht dazu. Im Beispiel der **Fig. 7** schließen die linienförmigen Strukturelemente 6 mit der Richtung 10 einen Winkel von 45 Grad ein.

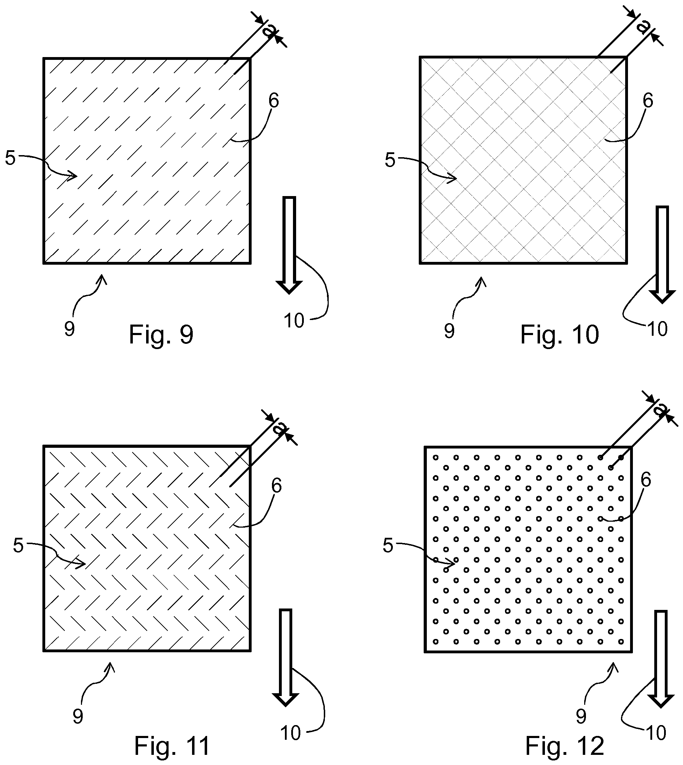

**Fig. 9 bis 12** zeigen in Draufsicht weitere Beispiele für eine Oberflächenstruktur 5, wie sie auf einer erfindungsgemäßen Komponente 1, 2, 3 ausgeformt sein kann. Jede Oberflächenstruktur 5 besteht aus einer Vielzahl von Strukturelementen 6. Es ist hier jeweils nur ein Ausschnitt 9 einer Komponente 1, 2, 3 gezeigt, wobei es unerheblich ist, ob es sich bei der Komponente um einen Zylinder 1, einen Kolben 2 oder eine Dichtung 3 handelt. Der Pfeil zeigt jeweils die bereits in **Fig. 1A und 1B** gezeigte Richtung 10 an.

In den in den **Fig. 9 bis 12** gezeigten Beispielen sind die Strukturelemente 6 regelmäßig angeordnet, die Oberflächenstrukturen 5 sind jeweils durch einen Abstand a zwischen benachbarten Strukturelementen 6 gekennzeichnet. Im Beispiel der **Fig. 9** sind die Strukturelemente 6 linienförmig, etwa linienförmige Erhöhungen oder Eintiefungen, jedoch im Gegensatz zum Beispiel der **Fig. 7** keine durchgehenden Linien. Die Strukturelemente schließen mit der Richtung 10 einen Winkel im Bereich 30 Grad bis 60 Grad ein. Im Beispiel der **Fig. 10** bilden die Strukturelemente 6 zwei Gruppen von linienförmigen Strukturelementen, welche sich unter einem rechten Winkel schneiden und mit der Richtung 10 einen Winkel von 45 Grad einschließen. In Beispiel der **Fig. 11** bilden die Strukturelemente 6 zwei Gruppen von linienförmigen Strukturelementen, welche unter einem rechten Winkel zueinander angeordnet sind und mit der Richtung 10 einen Winkel von 45 Grad einschließen. **Fig. 12** zeigt eine weitere regelmäßige Anordnung von punktförmigen Strukturelementen 6.

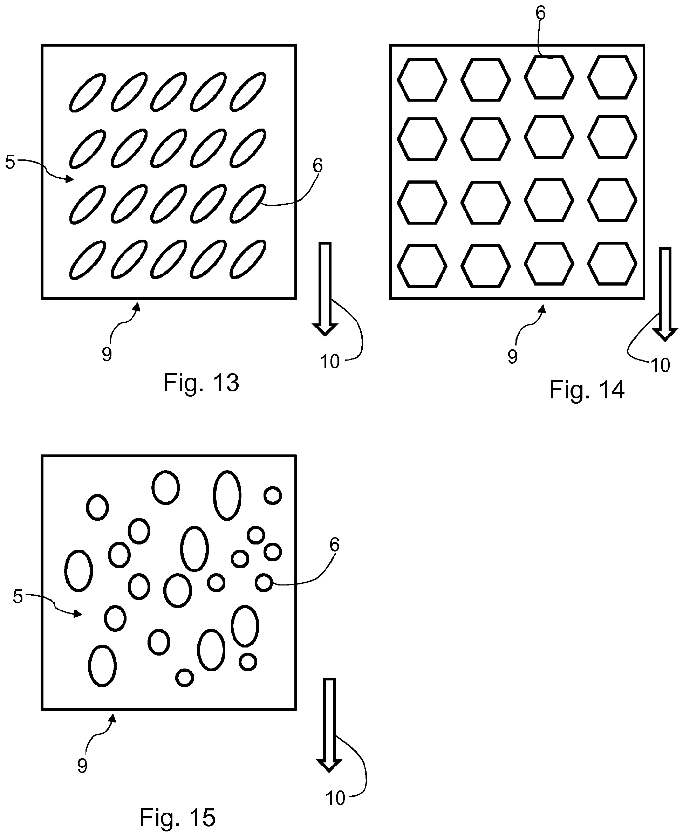

**Fig. 13 bis 15** zeigen in Draufsicht weitere Beispiele für eine Oberflächenstruktur 5, wie sie auf einer erfindungsgemäßen Komponente 1, 2, 3 ausgeformt sein kann. Jede Oberflächenstruktur besteht aus einer Vielzahl von Strukturelementen 6. Es ist hier jeweils nur ein Ausschnitt 9 einer Komponente 1, 2, 3 gezeigt, wobei es unerheblich ist, ob es sich bei der Komponente um einen Zylinder 1, einen Kolben 2 oder eine Dichtung 3 handelt. Der Pfeil zeigt jeweils die bereits in **Fig. 1A und 1B** gezeigte Richtung 10 an.

**Fig. 13** zeigt regelmäßig angeordnete Strukturelemente 6 von elliptischem Querschnitt. **Fig. 14** zeigt regelmäßig angeordnete Strukturelemente 6 von polygonalem, hier insbesondere hexagonalem, Querschnitt. **Fig. 15** zeigt unregelmäßig angeordnete Strukturelemente 6, die sich außerdem noch in Größe und Form unterscheiden.

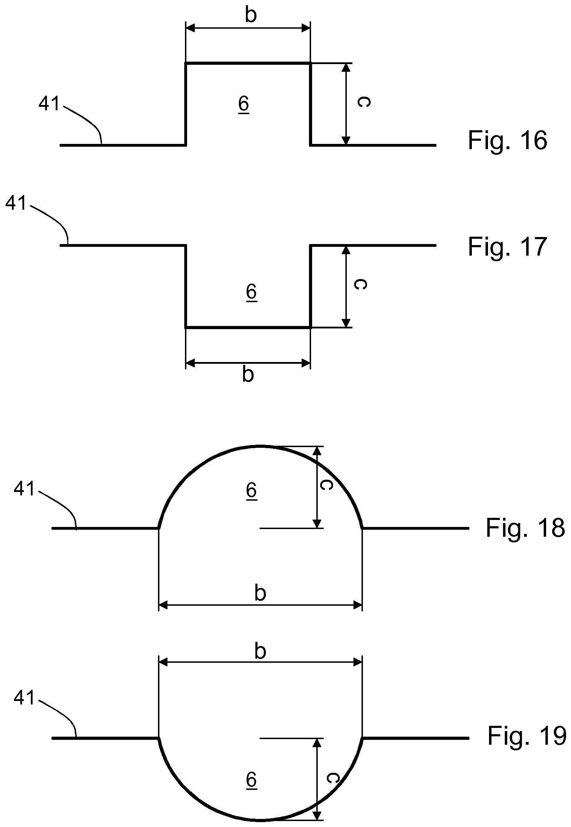

**Fig. 16 bis 19** zeigen Beispiele für Schnittansichten durch Strukturelemente 6, wie sie in Oberflächenstrukturen auf erfindungsgemäßen Komponenten 1, 2, 3 vorkommen können. Die Strukturelemente 6 sind durch ein Breite b gekennzeichnet. In den Beispielen der **Fig. 16** und **Fig. 18** bilden die Strukturelemente 6 Erhöhungen gegenüber einem Referenzniveau 41 der Komponente 1, 2, 3 und sind durch eine Höhe c bezogen auf dieses Referenzniveau 41 gekennzeichnet. In den Beispielen der **Fig. 17** und **Fig. 19** bilden die Strukturelemente 6 Eintiefungen gegenüber einem Referenzniveau 41 der Komponente 1, 2, 3, und sind durch eine Tiefe c bezogen auf dieses Referenzniveau 41 gekennzeichnet. Das Referenzniveau 41 ist beispielsweise durch eine Oberfläche der jeweiligen Komponente 1, 2, 3 im Bereich zwischen den Strukturelementen 6 gegeben. Die Beispiele der **Fig. 16** und **Fig. 17** zeigen Schnittansichten durch linienförmige Strukturelemente 6 mit rechteckigem Querschnitt, die Beispiele der **Fig. 18** und **Fig. 19** zeigen Schnittansichten durch Strukturelemente 6 mit kreisförmigem Grundriss und gerundetem Querschnitt.

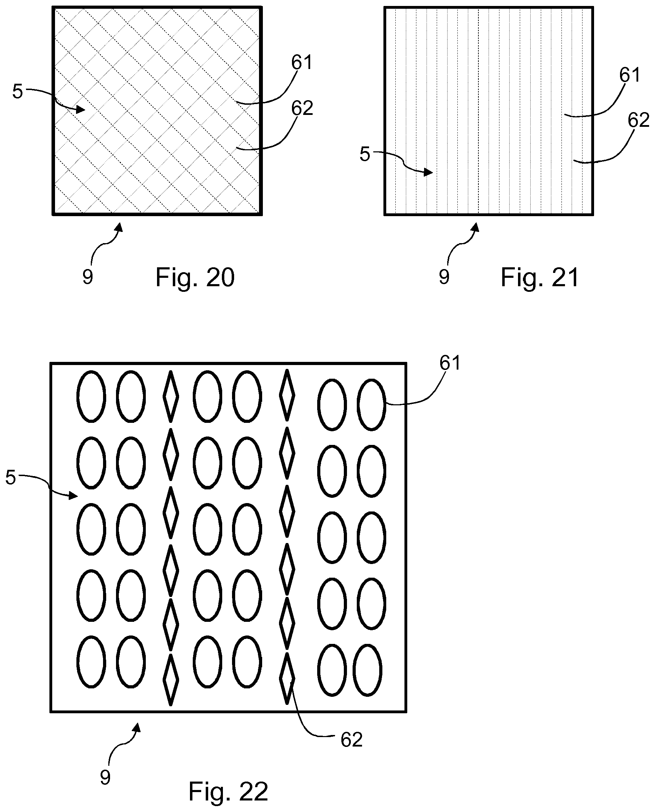

**Fig. 20** und **Fig. 21** zeigen in Draufsicht jeweils ein Beispiel einer erfindungsgemäßen Komponente 1, 2, 3. Es ist jeweils nur ein Ausschnitt 9 der Komponente 1, 2, 3 gezeigt. Die Oberflächenstruktur 5 umfasst hier zwei Gruppen von Strukturelementen, eine Gruppe erster Strukturelemente 61 und eine Gruppe zweiter Strukturelemente 62. Die ersten Strukturelemente 61 sind untereinander gleichartig. Die zweiten Strukturelemente 62 sind untereinander gleichartig. Die ersten Strukturelemente 61 unterscheiden sich von den zweiten Strukturelementen 62. Beispielsweise können die ersten Strukturelemente 61 die Form unterbrochener Linien haben, während die zweiten Strukturelemente 62 die Form durchgezogener Linien haben. In **Fig. 20** sind die ersten Strukturelemente 61 und die zweiten Strukturelemente 62 so angeordnet, dass sich die Strukturelemente gegenseitig durchdringen. In **Fig. 21** sind die ersten Strukturelemente 61 parallel zu den zweiten Strukturelementen 62 angeordnet. Eine Anordnung wie in **Fig. 21** begünstigt eine Verhakung bei Gebrauch, so dass eine Anordnung gemäß **Fig. 20** bevorzugt ist.

**Fig. 22** zeigt in Draufsicht ein weiteres Beispiel einer erfindungsgemäßen Komponente 1, 2, 3. Es ist nur ein Ausschnitt 9 der Komponente 1, 2, 3 gezeigt. Die Oberflächenstruktur 5 umfasst hier wie in den Beispielen der **Fig. 20 und 21** zwei Gruppen von Strukturelementen, eine Gruppe erster Strukturelemente 61 und eine Gruppe zweiter Strukturelemente 62. Die ersten Strukturelemente 61 sind untereinander gleichartig. Die zweiten Strukturelemente 62 sind untereinander gleichartig. Die ersten Strukturelemente 61 unterscheiden sich von den zweiten Strukturelementen 62. Die ersten Strukturelemente 61 haben einen elliptischen Grundriss, die zweiten Strukturelemente 62 haben einen polygonalen, hier rautenförmigen Grundriss.

### Bezugszeichenliste:

- 1: Zylinder
- 2: Kolben
- 3: Dichtung
- 4: Bereich (einer Komponente)
- 41: Referenzniveau
- 5: Oberflächenstruktur
- 6: Strukturelement
- 61: erstes Strukturelement
- 62: zweites Strukturelement
- 7: Mantel (des Zylinders)
- 8: Innenseite (des Mantels)
- 9: Ausschnitt (einer Komponente)
- 10: Richtung
- 101: Spritze
- 102: Spritze
- a: Abstand
- b: Breite
- c: Höhe bzw. Tiefe

## Patentansprüche

1. Komponente (1, 2, 3) für eine Spritze (101, 102) umfassend
eine Oberflächenstruktur (5), als welche ein zusammenhängender Bereich (4) der Komponente (1, 2, 3) ausgeformt ist, und welche dazu ausgebildet ist, durch ihre Form in der Spritze (101, 102) reibungsreduzierend zu wirken,
wobei die Komponente ein Zylinder (1) für eine Spritze (101, 102) ist, und die Oberflächenstruktur (5) sich an einer Innenseite (8) eines Mantels (7) des Zylinders (1) befindet, oder
wobei die Komponente ein Kolben (2) für eine Spritze (101, 102) ist, und die Oberflächenstruktur (5) so an dem Kolben (2) angeordnet ist, dass sie in der Spritze (101, 102) einem Mantel (7) eines Zylinders (1) für die Spritze (101, 102) gegenüberliegt, oder
wobei die Komponente eine Dichtung (3) für einen Kolben (2) einer Spritze (102) ist, und die Oberflächenstruktur (5) so an der Dichtung (3) angeordnet ist, dass sie in der Spritze (102) einem Mantel (7) eines Zylinders (1) für die Spritze (102) gegenüberliegt,
wobei die Oberflächenstruktur (5) eine Vielzahl von Strukturelementen (6) aufweist, welche sowohl entlang einer Richtung (10) als auch entlang einer anderen Richtung senkrecht zu der Richtung (10) angeordnet sind, wobei die Strukturelemente (6) unmittelbar auf dem zusammenhängenden Bereich (4) der Komponente (1, 2, 3) aus dem Material der Komponente (1, 2, 3) ausgeformt sind.

2. Komponente (1, 2, 3) nach Anspruch 1, wobei die Strukturelemente (6) gleichartig oder unterschiedlich sind.

3. Komponente (1, 2, 3) nach Anspruch 2, wobei jedes Strukturelement (6) von einer der folgenden Arten ist: linienförmige Eintiefung, linienförmige Erhöhung, punktförmige Eintiefung, punktförmige Erhöhung, Eintiefung mit elliptischem Grundriss, Erhöhung mit elliptischem Grundriss, Eintiefung mit polygonalem Grundriss, Erhöhung mit polygonalem Grundriss.

4. Komponente (1, 2, 3) nach einem der Ansprüche 2 oder 3, wobei die Strukturelemente (6) regelmäßig oder unregelmäßig angeordnet sind.

5. Komponente (1, 2, 3) nach einem der Ansprüche 2 bis 4, wobei ein Abstand (a) zwischen Strukturelementen (6) im Bereich 10 µm bis 1 mm liegt.

6. Komponente (1, 2, 3) nach einem der Ansprüche 2 bis 5, wobei eine Breite (b) eines Strukturelements (6) im Bereich 10 µm bis 100 µm liegt.

7. Komponente (1, 2, 3) nach einem der Ansprüche 2 bis 6, wobei eine Höhe oder eine Tiefe (c) eines Strukturelements (6) im Bereich 1 µm bis 100 µm liegt,

8. Komponente (1, 2, 3) nach einem der Ansprüche 2 bis 7, wobei die Strukturelemente (6) auf der Komponente (1, 2, 3) so angeordnet sind, dass sie bezüglich einer Bewegungsrichtung (10) des Kolbens (2) im Zylinder (1) der Spritze (101, 102) parallel oder senkrecht oder in einem Winkel zwischen 30 Grad und 60 Grad liegen.

9. Komponente (1, 2, 3) nach einem der Ansprüche 2 bis 8, wobei die Oberflächenstruktur (5) mindestens zwei Gruppen von Strukturelementen (6) umfasst, wobei die Strukturelemente (6) innerhalb einer Gruppe gleichartig sind, und wobei sich die Strukturelemente (6) zweier Gruppen hinsichtlich wenigstens eines der folgenden Merkmale unterscheiden: Form, Breite (b), Höhe (c), Tiefe (c), Ausrichtung bezüglich einer Bewegungsrichtung (10) des Kolbens (2) im Zylinder (1) der Spritze (101, 102).

10. Spritze (101, 102) mit mindestens einer Komponente (1, 2, 3) gemäß einem der Ansprüche 1 bis 9.

11. Spritze (101, 102) nach Anspruch 10, mit zwei Komponenten (1, 2, 3) gemäß einem der Ansprüche 1 bis 9, wobei sich eine zusätzliche reibungsreduzierende Wirkung aus dem Zusammenwirken der jeweiligen Oberflächenstrukturen (5) der Komponenten (1, 2, 3) ergibt.

## Claims

1. Component (1, 2, 3) for a syringe (101, 102), comprising
a surface structure (5) as which a continuous region (4) of the component (1, 2, 3) is formed and which is designed to act in a friction-reducing manner by its shape in the syringe (101, 102),
wherein the component is a cylinder (1) for a syringe (101, 102), and the surface structure (5) is located on an inside (8) of a shell (7) of the cylinder (1), or
wherein the component is a plunger (2) for a syringe (101, 102), and the surface structure (5) is arranged on the plunger (2) so that in the syringe (101, 102) it is opposite a shell (7) of a cylinder (1) for the syringe (101, 102), or
wherein the component is a seal (3) for a plunger (2) of a syringe (102), and the surface structure (5) is arranged on the seal (3) so that in the syringe (102) it is opposite a shell (7) of a cylinder (1) for the syringe (102),
wherein the surface structure (5) comprises a plurality of structural elements (6), which are arranged both along one direction (10) and along another direction perpendicular to the direction (10),
wherein the structural elements (6) are formed directly on the continuous region (4) of the component (1, 2, 3) from the material of the component (1, 2, 3).

2. Component (1, 2, 3) according to claim 1, wherein the structural elements (6) are identical or different.

3. Component (1, 2, 3) according to claim 2, wherein each structural element (6) is one of the following types: linear recess, linear elevation, punctiform recess, punctiform elevation, recess with elliptical layout, elevation with elliptical layout, recess with polygonal layout, elevation with polygonal layout.

4. Component (1, 2, 3) according to anyone of claims 2 or 3, wherein the structural elements (6) are arranged in a regular or irregular manner.

5. Component (1, 2, 3) according to anyone of claims 2 to 4, wherein a spacing (a) between structural elements (6) is in the range of 10 µm to 1 mm.

6. Component (1, 2, 3) according to anyone of claims 2 to 5, wherein a width (b) of a structural element (6) is in the range of 10 µm to 100 µm.

7. Component (1, 2, 3) according to anyone of claims 2 to 6, wherein a height or depth (c) of a structural element (6) is in the range of 1 µm to 100 µm.

8. Component (1, 2, 3) according to anyone of claims 2 to 7, wherein the structural elements (6) are arranged on the component (1, 2, 3) so as to be parallel or perpendicular or at an angle between 30 degrees and 60 degrees with respect to a direction of movement (10) of the plunger (2) in the cylinder (1) of the syringe (101, 102).

9. Component (1, 2, 3) according to anyone of claims 2 to 8, wherein the surface structure (5) comprises at least two groups of structural elements (6), wherein the structural elements (6) within a group are similar, and wherein the structural elements (6) of two groups differ with respect to at least one of the following features: shape, width (b), height (c), depth (c), orientation with respect to a direction of movement (10) of the plunger (2) in the cylinder (1) of the syringe (101, 102).

10. Syringe (101, 102) having at least one component (1, 2, 3) according to anyone of claims 1 to 9.

11. Syringe (101, 102) according to claim 10, comprising two components (1, 2, 3) according to anyone of claims 1 to 9, wherein an additional friction-reducing effect results from the interaction of the respective surface structures (5) of the components (1, 2, 3).

## Revendications

1. Composant (1, 2, 3) pour une seringue (101, 102), comprenant
une structure de surface (5) sous la forme de laquelle une zone continue (4) du composant (1, 2, 3) est formée et qui est adaptée par sa forme à avoir un effet de réduction du frottement dans la seringue (101, 102),
lequel composant est un cylindre (1) pour une seringue (101, 102) et la structure de surface (5) se trouve sur un côté intérieur (8) d'une enveloppe (7) du cylindre (1), ou
lequel composant est un piston (2) pour une seringue (101, 102) et la structure de surface (5) est disposée sur le piston (2) de telle sorte que, dans la seringue (101, 102), elle se trouve en face d'une enveloppe (7) d'un cylindre (1) de la seringue (101, 102), ou
lequel composant est un joint (3) pour un piston (2) d'une seringue (102) et la structure de surface (5) est disposée sur le joint (3) de telle sorte que, dans la seringue (102), elle se trouve en face d'une enveloppe (7) d'un cylindre (1) de la seringue (102),
la structure de surface (5) présentant une pluralité d'éléments structurels (6) qui sont disposés aussi bien dans une direction (10) que dans une autre direction perpendiculaire à la direction (10),
les éléments structurels (6) étant formés directement sur la zone continue (4) du composant (1, 2, 3) à partir du matériau du composant (1, 2, 3).

2. Composant (1, 2, 3) selon la revendication 1, dans lequel les éléments structurels (6) sont identiques ou différents.

3. Composant (1, 2, 3) selon la revendication 2, dans lequel chaque élément structurel (6) est de l'un des types suivants : renfoncement linéaire, élévation linéaire, renfoncement punctiforme, élévation punctiforme, renfoncement à plan elliptique, élévation à plan elliptique, renfoncement à plan polygonal, élévation à plan polygonal.

4. Composant (1, 2, 3) selon l'une des revendications 2 ou 3, dans lequel les éléments structurels (6) sont disposés de manière régulière ou irrégulière.

5. Composant (1, 2, 3) selon l'une des revendications 2 à 4, dans lequel une distance (a) entre les éléments structurels (6) se situe dans la plage de 10 µm à 1 mm.

6. Composant (1, 2, 3) selon l'une des revendications 2 à 5, dans lequel une largeur (b) d'un élément structurel (6) se situe dans la plage de 10 µm à 100 µm.

7. Composant (1, 2, 3) selon l'une des revendications 2 à 6, dans lequel une hauteur ou une profondeur (c) d'un élément structurel (6) se situe dans la plage de 1 µm à 100 µm.

8. Composant (1, 2, 3) selon l'une des revendications 2 à 7, dans lequel les éléments structurels (6) sont disposés sur le composant (1, 2, 3) de manière à être parallèles, perpendiculaires ou selon un angle compris entre 30 degrés et 60 degrés par rapport à une direction de déplacement (10) du piston (2) dans le cylindre (1) de la seringue (101, 102).

9. Composant (1, 2, 3) selon l'une des revendications 2 à 8, dans lequel la structure de surface (5) comprend au moins deux groupes d'éléments structurels (6), dans lequel les éléments structurels (6) à l'intérieur d'un groupe sont du même type et dans lequel les éléments structurels (6) de deux groupes diffèrent en ce qui concerne au moins une des caractéristiques suivantes : forme, largeur (b), hauteur (c), profondeur (c), orientation par rapport à une direction de déplacement (10) du piston (2) dans le cylindre (1) de la seringue (101, 102).

10. Seringue (101, 102) comportant au moins un composant (1, 2, 3) selon l'une des revendications 1 à 9.

11. Seringue (101, 102) selon la revendication 10, comportant deux composants (1, 2, 3) selon l'une des revendications 1 à 9, dans laquelle un effet supplémentaire de réduction du frottement résulte de l'interaction des structures de surface respectives (5) des composants (1, 2, 3).
